# EUROPÄISCHE PATENTANMELDUNG

(11) **EP 3 763 807 A1**
(43) Veröffentlichungstag der Anmeldung: **13.01.2021**
(21) Anmeldenummer: 19186043.6
(22) Anmeldetag: 12.07.2019
(51) Int. Cl.: C12M 1/00, C12M 1/12

(54) **MULTI-CONNECTOR-PORT**

(71) Anmelder: Eppendorf AG, 22339 Hamburg (DE)
(72) Erfinder: Selzer, Sebastian, 52074 Aachen (DE); Rix, Karl, 42555 Velbert (DE); Streule, Wolfgang, 52445 Titz-Rödingen (DE)
(74) Vertreter: Eisenführ Speiser

(57) **Zusammenfassung**

Die Erfindung betrifft einen Multi-Connector-Port umfassend einen Luftanschluss für sterile Luft sowie mindestens einen an einem Verbindungsstück angeordneten Zugang und mindestens eine am Verbindungsstück angeordnete Leitung, wobei der Zugang eine selbstschließende Membran umfasst.

## Beschreibung

Die Erfindung betrifft einen Multi-Connector-Port sowie ein Verfahren zum Befüllen oder Entnehmen über einen Multi-Connector-Port.

In Laboren, insbesondere in Laboren für biologische Verfahren, besteht ein zunehmendes Bedürfnis nach Automatisierungslösungen, die die Reproduzierbarkeit erhöhen, mit kleinen Volumina arbeiten können und gleichzeitig flexibel einsetzbar sind. Derzeit existieren zwei Gruppen von automatisierten Lösungen für Bioreaktorsysteme: Autosampler und automatisierte Pipettierungssysteme für kleine Volumina oder eigenständige Einzellösungen. Autosampler können wegen fehlender standardisierter Anschlüsse nur in speziellen, nach außen abgeschlossenen aseptischen Systemen eingesetzt werden, darüber hinaus sind sie auf eine Probenentnahme beschränkt und es verbleiben im Sampler zumeist Totvolumina. Auch Verweilzeiten im Sampler können im Bereich der biologischen Prozesse die Probe beeinflussen. Automatisierte Pipettierungssysteme für kleine Volumina oder eigenständige Einzellösungen haben den Nachteil, dass sie in der Regel nur für bestimmte Bioreaktoren oder Behältnisse geeignet sind und die Arbeiten mit diesen Lösungen in Arbeitsstationen stattfinden müssen. In der Regel ist mit diesen Systemen auch kein Arbeiten mit single-use Bioreaktoren möglich.

Der Erfindung liegt daher die Aufgabe zugrunde, eine verbesserte Lösung bereitzustellen, die die genannten Probleme adressiert.

Gemäß einem ersten Aspekt der Erfindung wird diese Aufgabe gelöst durch einen Multi-Connector-Port umfassend einen Luftanschluss für sterile Luft sowie mindestens ein Verbindungsstück, mindestens einen am Verbindungsstück angeordneten Zugang sowie mindestens eine am Verbindungsstück angeordnete Leitung, wobei der Zugang eine selbstschließende Membran umfasst.

Der Erfindung liegt die Erkenntnis zugrunde, dass automatisierte Zugaben und Entnahmen insbesondere in Bioreaktoren und Gefäße, ohne die Notwendigkeit in sterilen Arbeitsstationen oder abgeschlossenen Systemen zu arbeiten und für eine Vielzahl von Ausführungsformen von Bioreaktoren und Gefäßen, möglich sind, wenn die Zugabe- oder Entnahmestelle vom Reaktor oder Gefäß entkoppelt wird. Hierzu wird zwischen Bioreaktor und Liquid-Handling Einrichtung ein Multi-Connector-Port eingesetzt, der mit dem Bioreaktor über mindestens eine Leitung steril und dicht verbunden werden kann und der Zugänge für die Liquid-Handling Einrichtung bereitstellt, die von Spitzen der Einrichtung unter Abschluss nach außen penetrierbar sind. Unter Spitzen der Einrichtung werden neben spitzen Hohlnadeln auch stumpfere Spitzen wie Pipettenspitzen oder männliche Verbindungsstücke verstanden. Penetrieren meint im Rahmen dieser Erfindung neben Durchstechen auch das Aufdrücken selbstschließender Membranen, wie beispielsweise in swabable valves, auch als abwischbare Ventile oder nadelfreie Membranventile bezeichnet, durch stumpfe Spitzen, beispielsweise männliche Verbindungsstücke.

Die Erfindung ermöglicht also automatisiertes, steriles Arbeiten in nicht-sterilen Umgebungen bei der Befüllung mit oder der Entnahme von Materialien beispielsweise in Bioreaktoren. Es können somit sowohl bestehende Handlingsysteme in nicht-steriler Umgebung als auch bestehende Bioreaktoren oder Gefäße zusammen mit dem Multi-Connector-Port eingesetzt werden, für eine kontaminationsfreie Entnahme oder Zugabe.

Darüber hinaus schließt die Erfindung die Erkenntnis ein, dass Totvolumina oder Kontaminationen durch vorherige Zu- oder Entnahmevorgänge verhindert oder minimiert werden können, wenn der Multi-Connector-Port über einen Anschluss für sterile Luft gespült werden kann, so dass im Verbindungsstück des Multi-Connector-Ports oder in der Leitung verbliebene Flüssigkeit in den Reaktor oder das Gefäß befördert werden kann. Somit kann auch mit sehr kleinen Proben- oder Zugabevolumina gearbeitet werden, da diese vollständig ausgenutzt werden können. Der erfindungsgemäße Multi-Connector-Port und dessen Verwendung stellen also eine Lösung bereit, die flexibel eingesetzt werden kann, keine sterile Umgebung erfordert und Totvolumina vermeidet.

Die Erfindung schließt darüber hinaus die Erkenntnis ein, dass eine Vielzahl von Arbeitsschritten mit dem erfindungsgemäßen Multi-Connector-Port möglich wird, die bei bestehenden Systemen nicht in einer Lösung integrierbar waren. So können mit dem Multi-Connector-Port beispielsweise Probenentnahme aus einem Bioreaktor, Probenabgabe in Mikroreaktionsgefäße für nachfolgende Analysen, Probenabgabe in einen Auffangbehälter, Probenabgabe in ein automatisiertes System, wie beispielsweise ein Analysegerät, Ausbluten eines Bioreaktors (Entfernung von Zellen aus dem Bioreaktor, um die Zellkonzentration zu steuern), Zugabe eines Mediums oder eines Medien-Cocktails beispielsweise in einen Bioreaktor realisiert werden. Des Weiteren ist es nunmehr möglich Medien oder Medien-Cocktails für in den Bioprozess automatisiert und zeitgesteuert zuzuführen.

In einer Ausführungsform umfasst der Luftanschluss eine weitere selbstschließende Membran und einen sterilen Filter, insbesondere einen sterilen Filter mit einem Porendurchmesser kleiner oder gleich 0,22 µm. Über einen derart ausgestalteten Luftanschluss kann sterile Luft einfach über eine Spitze zugegeben werden, ohne dass konstruktiv aufeinander abgestimmte Anschlüsse am Multi-Connector-Port und der sterilen Luftzuführung vorhanden sein müssen. Alternativ ist auch eine Ausgestaltung des Luftanschlusses mit gängigen Luftanschlusssystemen möglich.

Der Luftanschluss kann dabei entweder so ausgeführt sein, dass über ihn sterile Luft in eine Spitze, beispielsweise eines Handlingsystems gezogen werden kann und dann von der Spitze in den mindestens einen Zugang abgegeben wird, der Luftanschluss ist dann separat und fluidisch nicht mit dem mindestens einen Zugang verbunden oder der Luftanschluss ist direkt fluidisch mit dem mindestens einen Verbindungsstück und damit mit dem mindestens einen Zugang verbunden.

In einer Ausführungsform hat daher eine der selbstschließenden Membran abgewandte Seite des sterilen Filters Kontakt zur Außenluft. Wird in dieser Ausführungsform eine Spitze durch die selbstschließende Membran eingeführt und mit dieser Außenluft über den sterilen Filter angesaugt, so steht die hernach in einem zur Spitze gehörenden Reservoir vorhandene sterile Luft für eine Applikation in den mindestens einen Zugang zur Verfügung, somit kann vergleichsweise einfach sterile Luft für eine Spülung bereitgestellt werden, ohne dass sterile Luft vorgehalten werden muss. Alternativ kann auch ein Luftanschluss eines gängigen Luftanschlusssystems, in dem sterile Luft vorhanden ist, vorgesehen sein und diese über die Spitze in das Reservoir gezogen werden.

In alternativen Ausführungsformen ist der Luftanschluss fluidisch mit dem Verbindungsstück verbunden, so dass der mindestens eine Zugang, der Luftanschluss und die mindestens eine Leitung miteinander über das mindestens eine Verbindungsstück fluidisch verbunden sind. So können über in den Luftanschluss abgegebene Luft Zugang, Verbindungsstück und Leitung gespült werden. Auch hier kann der Luftanschluss sowohl als Anschluss eines Luftanschlusssystems ausgebildet sein als auch eine weitere selbstschließende Membran und einen sterilen Filter umfassen, insbesondere einen sterilen Filter mit einem Porendurchmesser kleiner oder gleich 0,22 µm. In letzterem Fall wird Luft beispielsweise über eine Spitze durch die Membran appliziert und vom sterilen Filter gefiltert in das Verbindungsstück appliziert.

In einer Ausführungsform umfasst der Multi-Connector-Port mehrere am Verbindungsstück angeordnete Zugänge. Das Vorsehen mehrerer Zugänge erlaubt beispielsweise die gleichzeitige Zugabe verschiedener Medien aber auch die gleichzeitige Entnahme mehrerer Proben.

In einer weiteren Ausführungsform weist der Multi-Connector-Port an jedem Verbindungsstück genau einen Zugang und genau eine Leitung auf. In dieser Ausführungsform werden die über die Zugänge zugegebenen oder entnommene Medien vom jeweiligen Zugang bis zum verbundenen Gefäß, beispielsweise dem Bioreaktor, vollständig getrennt geführt. Damit können beispielsweise Vermischungen vor Eintritt in den Bioreaktor vermieden werden. Es ist insbesondere bevorzugt, wenn der Multi-Connector-Port genau zwei Verbindungsstücke aufweist, an denen jeweils ein Zugang und eine Leitung angeordnet sind. Weiterhin ist es bevorzugt, wenn ein Zugang für eine Zugabe von Medien und ein Zugang für eine Entnahme von Medien ausgebildet ist. In dieser Ausführungsform können Proben entnommen werden, ohne dass die Gefahr von Verfälschungen durch zuvor zugegebenen Medien besteht.

Der Multi-Connector-Port weist in einer Ausführungsform eine Vielzahl von Zugängen und Verbindungsstücken auf. Auch in dieser Ausführungsform ist nur ein Luftanschluss notwendig, dieser kann zur Spülung für alle Zugänge verwendet werden. Hier ist es insbesondere vorteilhaft, wenn der Luftanschluss derart ausgebildet ist, dass durch ihn sterile Luft in eine Spitze angesaugt werden kann. Diese Ausführungsform ist insbesondere vorteilhaft, da sie mit einer Vielzahl von Bioreaktoren gleichzeitig eingesetzt werden kann.

In weiteren Ausführungsformen sind der jeweilige Zugang und die jeweilige Leitung sowie der Luftanschluss über das jeweilige Verbindungsstück fluidisch verbunden. Damit ist eine Spülung über einen Luftanschluss in beiden Zugängen möglich, die Führung der Medien zwischen Gefäß und Zugang erfolgt aber getrennt voneinander, so dass Vermischungen oder Kontaminationen vermieden werden.

Vorteilhaft weist der Multi-Connector-Port eine Fixiervorrichtung auf. Über diese können beispielsweise Verbindungsstücke miteinander mechanisch verbunden sein für eine bessere Handhabbarkeit als geschlossenes Bauteil. Insbesondere ist es aber auch vorteilhaft eine Fixiervorrichtung einzusetzen, die zur Verbindung des Multi-Connector-Ports mit einem Handlingsystem, insbesondere einem Handlingroboter, ausgebildet ist. So kann der Multi-Connector-Port exakt positioniert und in Position gehalten werden und automatisierte Einrichtungen, wie ein Handlingroboter die Zugänge automatisiert nutzen.

Bevorzugt ist der Multi-Connector-Port sterilisierbar ausgeführt, wobei die Sterilisation bevorzugt über Autoklavieren, Bestrahlung oder mit Ethylenoxid erfolgen kann. So kann durch einfache Sterilisationsmethoden ein steriler Zugang über den Multi-Connector-Port realisiert werden, ohne dass Arbeiten in abgeschlossenen Systemen oder Arbeitsstationen notwendig sind.

In einer Ausführungsform des Multi-Connector-Ports weist die mindestens eine Leitung an ihrem dem Verbindungsstück abgewandten Ende einen gas- und flüssigkeitsdichten Anschluss auf. Über diesen Anschluss kann der Multi-Connector-Port einfach und sicher beispielsweise an die Kopfplatte eines Bioreaktors angeschlossen werden. Hier sind insbesondere standardisierte Anschlüsse, die mit möglichst vielen Behältern oder Kopfplatten kompatibel sind von Vorteil. Beispielsweise kann es sich um einen Luer-Lock-Anschluss handeln oder Schraubanschlüsse. In einer sehr einfachen Ausgestaltung ist die mindestens eine Leitung als einfacher Schlauch ausgeführt, der beispielsweise in einen Anschluss einer Kopfplatte eines Bioreaktors eingesteckt werden kann.

Es ist zusätzlich vorteilhaft, wenn der Multi-Connector-Port eine Kappe zum Abdecken des mindestens einen Zugangs und/oder des Luftanschlusses besitzt. Hierüber können Zugang und/oder Luftanschluss gegen Verunreinigungen geschützt werden, solange sie nicht in Benutzung sind.

Die selbstschließende Membran ist in einer Ausführungsform als durchstechbares Septum ausgebildet, also als ein Septum, dass auch bei Penetration außerhalb der Penetration undurchlässig ist und mit dem eindringenden Gegenstand einen dichten Abschluss bildet. Es ist insbesondere bevorzugt, wenn das durchstechbare Septum für Gase und Flüssigkeiten bis zu einem Druck von 0,5 bar undurchlässig ist, auch wenn das Septum von einer Hohlnadel oder einer Pipettenspitze, insbesondere von einer Hohlnadel oder Pipettenspitze mit einem Durchmesser unter 1,5 mm, penetriert wird und auch nach mehrfacher Penetration durch eine derartige Hohlnadel oder Pipettenspitze. Das durchstechbare Septum weist in einer Ausführungsform einen Schlitz, insbesondere einen Kreuzschlitz für die Penetration auf. Das Septum kann beispielsweise aus Silikon gefertigt sein, derartige Septen sind aus dem Stand der Technik bereits bekannt.

Der Zugang kann in einer alternativen Ausführungsform aber auch in als nadelfreies Membranventil umfassend die selbstschließende Membran ausgebildet sein, derartige Ventile sind beispielsweise aus US 5,368,801 A, US 7,9497,032 B2 oder WO 2013/158756 bekannt.

Gemäß einem zweiten Aspekt betrifft die Erfindung ein Verfahren zum Befüllen oder Entnehmen über einen Multi-Connector-Port nach einem der vorstehenden Ansprüche umfassend die Schritte
- Verbinden der mindestens einen Leitung des Multi-Connector-Ports mit einem Gefäß, insbesondere einem Bioreaktor
- Reinigen einer Außenfläche des mindestens einen Zugangs und/oder einer Außenfläche des Luftanschlusses, insbesondere durch Abwischen oder Spülen mit einem Reinigungsmittel umfassend Isopropanol.
- Penetrieren der selbstschließenden Membran des mindestens einen Zugangs mit einer Spitze, insbesondere in Form einer Hohlnadel, einer Pipettenspitze oder einem männlichen Verbindungsstück,
- Zugeben eines Mediums in das Gefäß oder Absaugen eines Mediums aus dem Gefäß über die Spitze
- Entfernen der Spitze
- Applikation von steriler Luft.

Das Verfahren gemäß dem zweiten Aspekt der Erfindung teilt die Vorteile des Multi-Connector-Ports gemäß dem ersten Aspekt der Erfindung.

Bevorzugt umfasst die Applikation von steriler Luft folgende Schritte:
- Penetrieren der weiteren selbstschließenden Membran des Luftanschlusses mit einer Spitze
- Ansaugen von Umgebungsluft durch den sterilen Filter über die Spitze in ein mit der Spitze verbundenes Reservoir
- Entfernen der Spitze aus der weiteren selbstschließenden Membran,
- Penetrieren der selbstschließenden Membran des mindestens einen Zugangs des Multi-Connector-Ports mit der Spitze
- Applizieren von Luft aus dem Reservoir in den Zugang.

Über dieses Verfahren wird es insbesondere im Zusammenspiel mit einem Handlingsystem einfach ermöglicht, sterile Luft für die Spülung zu verwenden und damit Totvolumina zu vermeiden, ohne dass sterile Luft vorgehalten werden muss. Damit kann das Arbeiten noch flexibler und unabhängiger von weiterer Infrastruktur erfolgen.

Alternativ erfolgt die Applikation von steriler Luft über den Luftanschluss, indem sterile Luft über den Luftanschluss in das mit dem Luftanschluss fluidisch verbundene mindestens eine Verbindungstück appliziert wird, wobei entweder sterile Luft von außen über den Luftanschluss zugeführt wird oder Luft über eine weitere selbstschließende Membran und einen sterilen Filter in das mindestens eine Verbindungsstück und die daran angeordnete mindestens eine Leitung sowie den mindestens einen Zugang appliziert wird.

In einer bevorzugten Ausführungsform erfolgt nach der Applikation steriler Luft eine abschließende Reinigung der Außenfläche des mindestens einen Zugangs und/oder der Außenfläche des Luftanschlusses. Die Reinigung erfolgt dabei bevorzugt mit Isopropanol oder ähnlichen Reinigungsflüssigkeiten und/oder einem Desinfektionsmittel. Der Reinigungsschritt vereinfacht es, mit dem Multi-Connector-Port auch außerhalb steriler Umgebungen zu arbeiten und dennoch das Befüllen und Entnehmen unter sterilen Bedingungen zu gewährleisten. Dabei ist es bevorzugt, wenn der der Multi-Connector-Port mindestens eine Kappe umfasst und diese vor dem Reinigen abgenommen wird und/oder nach der abschließenden Reinigung aufgesetzt wird.

Es ist insbesondere vorteilhaft, wenn das Zugeben eines Mediums über einen anderen Zugang und eine andere Leitung erfolgt als das Absaugen.

In einer Ausführungsform des Verfahrens, bei der die Spitze Teil eines Handlingsystems ist und das Verbindungsstück eine Fixiervorrichtung aufweist, wird der Multi-Connector-Port vor dem Reinigen in Relation zum Handlingsystem über die Fixiervorrichtung exakt positioniert. Darüber wird ein automatisiertes Befüllen und Entnehmen über das Handlingsystem erleichtert.

Gemäß einem dritten Aspekt betrifft die Erfindung ein System umfassend einen Multi-Connector-Port gemäß dem ersten Aspekt der Erfindung sowie ein Handlingsystem mit mindestens einer Spitze, insbesondere in Form einer Hohlnadel, einer Pipettenspitze oder einem männlichen Verbindungsstück. Ein solches System ermöglicht auch ohne sterile Umgebung automatisierte kontaminationsfreie Befüllung und Entnahme an Bioreaktoren oder anderen Gefäßen. Im Übrigen teilt auch das System gemäß dem dritten Aspekt der Erfindung die Vorteile des Multi-Connector-Ports sowie des Verfahrens gemäß der weiteren Aspekte der Erfindung.

Das Handlingsystem kann dabei insbesondere ein Handlingroboter oder ein automatisiertes Liquid-Handling-System sein.

In einer Ausführungsform umfasst das System weiter eine Kapseleinrichtung zur Kapselung der Spitze gegen Umgebungsluft. Mit Hilfe einer solchen Kapseleinrichtung können Entnahme oder Zugabe noch sicherer durchgeführt werden, da die Spitze hierüber nicht in Kontakt zur Umgebungsluft tritt und Kontaminationen somit noch besser vermieden werden können. Die Kapseleinrichtung kann insbesondere als eine Düseneinrichtung zur Umspülung der Spitze mit steriler Luft oder als eine selbstschließende Ummantelung ausgeführt sein.

Bei einer Düseneinrichtung zur Umspülung der Spitze mit steriler Luft ist mindestens eine Düse verbunden mit einem Anschluss für sterile Luft in der Umgebung der Spitze angeordnet und so auf die Spitze gerichtet, dass diese zumindest in der Länge, in der sie in den mindestens einen Zugang oder der Luftanschluss maximal eindringen kann, von der sterilen Luft umspült wird und keinen Kontakt zur Umgebungsluft hat. Es wird über die Düseneinrichtung somit ein steriler Luftvorhang etabliert. Die Umspülung mit steriler Luft kann dauerhaft erfolgen oder zumindest während eines Eindringens der Spitze bis zur vollständigen Penetration und so lange Spitze nicht penetriert.

Die selbstschließende Ummantelung umschließt die Spitze luftdicht, wenn diese frei ist, also nichts penetriert, sie wird bei einer Penetration durch die Spitze beispielsweise in eine selbstschließende Membran aufgedrückt und schließt sich beim Herausziehen wieder. Die selbstschließende Ummantelung, die beispielsweise aus Silikon gefertigt sein kann, weist dabei beispielsweise einen Kreuzschlitz auf, der sich über eine bestimmte Länge der Spitze erstreckt. Wird die Spitze nun in eine selbstschließende Membran gedrückt, wird auch die Ummantelung aufgedrückt, diese schließt dann zusammen mit der selbstschließenden Membran die Spitze gegen die Umgebungsluft ab. Sobald die Spitze zurückgezogen wird, legt sich die Ummantelung wieder um die Spitze. Die selbstschließende Ummantelung kann insbesondere in Form einer Kappe, beispielsweise einer Silikonkappe mit einem Kreuzschlitz, ausgeführt sein.

In einer weiteren Alternative umfasst der Luftanschluss und oder der mindestens eine Zugang eine Kappe, die automatisiert auf die Spitze aufgesetzt und abgenommen werden kann.

Nachfolgend werden weitere Ausführungsformen beispielhaft anhand der beiliegenden Figuren erläutert. Es zeigen:
Fig. 1: eine Ausführungsform eines Multi-Connector-Ports gemäß dem ersten Aspekt der Erfindung;
Fig. 2: eine weitere Ausführungsform eines Multi-Connector-Ports gemäß dem ersten Aspekt der Erfindung;
Fig. 3a: eine weitere Ausführungsform eines Multi-Connector-Ports gemäß dem ersten Aspekt der Erfindung;
Fig. 3b: eine weitere Ausführungsform eines Multi-Connector-Ports gemäß dem ersten Aspekt der Erfindung;
Fig. 4: eine weitere Ausführungsform eines Multi-Connector-Ports gemäß dem ersten Aspekt der Erfindung;
Fig. 5: eine Ausführungsform eines Verfahrens gemäß dem zweiten Aspekt der Erfindung;
Fig. 6 eine Ausführungsform eines Systems gemäß dem dritten Aspekt der Erfindung;
Fig. 7 in einer Detailansicht Teile einer Ausführungsform eines Systems gemäß dem dritten Aspekt der Erfindung;
Fig. 8 in einer Detailansicht Teile einer Ausführungsform eines Systems gemäß dem dritten Aspekt der Erfindung in zwei Zuständen.

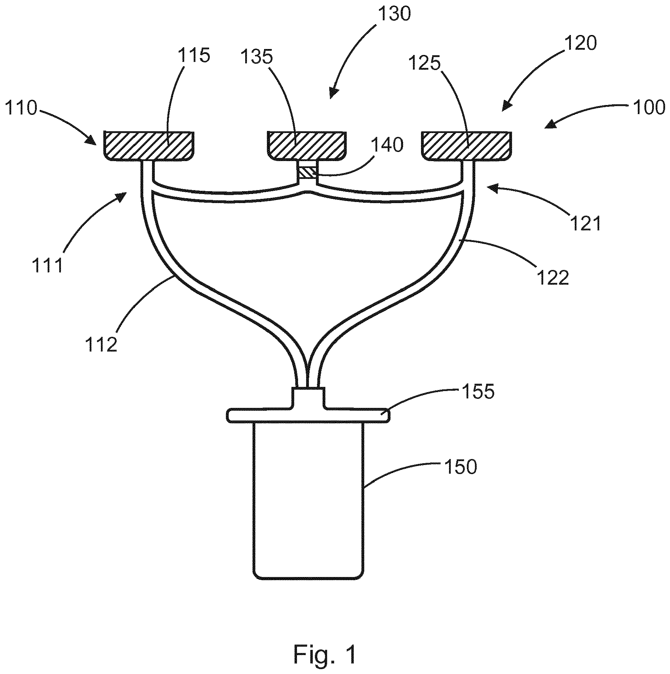

Fig.1 zeigt eine Ausführungsform eines Multi-Connector-Ports 100 gemäß dem ersten Aspekt der Erfindung. In der gezeigten Ausführungsform weist der Multi-Connector-Port 100 zwei Zugänge 110, 120 auf. Jeder dieser Zugänge 110, 120 ist an einem Verbindungsstück 111, 121 angeordnet. Des Weiteren ist am jeweiligen Verbindungsstück 111, 121 je eine Leitung 112, 122 angeordnet. Jeder der Zugänge 110, 120 weist eine selbstschließende Membran 115, 125 auf. In der hier gezeigten Ausführungsform ist die selbstschließende Membran 115, 125 jedes Zugangs 110, 120 als durchstechbares Septum ausgebildet. Das Septum kann dabei beispielsweise ein handelsübliches Silikonseptum sein, dieses kann von einer Spitze beispielsweise in Form einer Hohlnadel oder einen Pipettenspitze penetriert werden und schließt dennoch nach außen dicht ab. In bevorzugten Ausführungsformen ist das durchstechbare Septum mit einem Schlitz, insbesondere einem Kreuzschlitz ausgebildet, durch den die Spitze dringen kann, sodass sich die Wände des Schlitzes dicht an der Spitze anlegen und damit das Lumen unter dem Septum nach außen hin dicht abschließen. In alternativen Ausführungsformen, die hier nicht gezeigt sind, können die Zugänge 110, 120 auch als nadelfreie Membranventile ausgebildet sein. Neben den Zugängen 110, 120 weist der Multi-Connector-Port 100 einen Luftanschluss 130 auf, der in der gezeigten Ausführungsform ebenfalls eine weitere selbstschließende Membran 135 umfasst sowie einen sterilen Filter 140. Der sterile Filter weist in der gezeigten Ausführungsform einen Porendurchmesser kleiner oder gleich 0,22 µm auf. In der gezeigten Ausführungsform ist der Luftanschluss 130 über die Verbindungsstücke 111, 121 mit den Leitungen 112, 122 und den Zugängen 110, 120 fluidisch verbunden. Mit dieser Ausführungsform des Luftanschlusses ist eine Spülung der Verbindungsstücke 111, 121 sowie der Leitungen 112, 122 mit steriler Luft einfach zu realisieren, indem Luft über eine Spitze durch die weitere selbstschließende Membran eingebracht wird und vom sterilen Filter 140 gefiltert wird. Somit muss keine sterile Luft mit entsprechenden Anschlussstücken für deren Aufbewahrung bereitgehalten werden, die dann an den Multi-Connector-Port angeschlossen werden kann. Alterativ ist es auch möglich, standardisierte Luftanschlüsse für Sterilluft am Multi-Connector-Port vorzusehen. Die Leitungen 112, 122 führen hier zur Kopfplatte 155 eines Bioreaktors 150, für dessen Befüllen und Entnehmen der Multi-Connector-Port 100 hier eingesetzt wird. Der Multi-Connector-Port 100 kann aber auch für eine Vielzahl anderer Gefäße und Anschlussmöglichkeiten eingesetzt werden. Er ist flexibel mit anderen Systemen kombinierbar. Dazu können an den Leitungen 112, 122 spezielle Anschlüsse für eine dichte Verbindung mit dem jeweiligen Gefäß vorhanden sein. Die Leitung 112, 122 können aber auch als einfache Schläuche ausgebildet sein, die in beispielsweise vorhandene Septen an Kopfplatten eingeführt werden. Der Multi-Connector-Port 100 ermöglicht ein einfaches und sicheres Befüllen und Entnehmen für eine Vielzahl von Gefäßen, indem die Zugabe- bzw. Entnahmestelle vom Gefäß selbst entkoppelt wird. Die Zugabe oder Entnahme erfolgt über die Zugänge 110, 120. In der hier gezeigten Ausführungsform ist es insbesondere vorteilhaft, wenn einer der Zugänge, hier der Zugang 110, nur für Zugaben und der andere Zugang, hier Zugang 120, nur für Entnahmen verwendet wird. Damit kann sichergestellt werden, dass aus dem Gefäß entnommene Proben nicht von zuvor über den Zugang 110 und sein Verbindungsstück 111 sowie die Leitung 112 zugegebenen Medien verunreinigt wird. Der Zugang 120 sowie sein Verbindungsstück 121 und die Leitung 122 sind fluidisch vom Zugang 110 abgetrennt. Beide Teile des Multi-Connector-Ports sind lediglich über den Luftanschluss miteinander verbunden, über den keine Flüssigkeiten ausgetauscht werden. Der Luftanschluss 130 dient, wie bereits erwähnt, zum Spülen der Verbindungsstücke und Leitungen, sodass etwaige in den Verbindungsstücken 111, 121 oder Leitungen 112, 122 vorhandene Medien durch die Zugabe von steriler Luft in den Reaktor gepresst werden. Zuvor zugegebene Medien werden also vollständig an den Reaktor weitergegeben und es verbleiben kaum oder keine Totvolumina innerhalb des Verbindungsstücks oder der Leitung. Darüber kann sichergestellt werden, dass die zuvor eingestellte Menge an Medium auch tatsächlich den Bioreaktor oder das sonstige Gefäß erreicht. Im Falle des Zugangs zur Entnahme wird über die Spülung mit steriler Luft entnommenes Medium wieder zurück in den Bioreaktor oder das jeweilige Gefäß gedrückt, sodass in der Entnahmeleitung kein Medium verbleibt, das dort über die Zeit anderen Bedingungen ausgesetzt wäre als im Reaktor selber. Damit wird auch für spätere Probeentnahmen sichergestellt, dass die Proben vollständig aus dem Innenraum des Bioreaktors oder des anderen Gefäßes stammen und nicht durch seit längerer Zeit in der Leitung oder dem Verbindungsstück verweilende Probenreste kontaminiert werden. Bevorzugt ist der gesamte Multi-Connector-Port 100 sterilisierbar ausgeführt. Es ist weiterhin vorteilhaft, wenn alle Außenflächen des Multi-Connector-Ports, insbesondere die Membran, einfach beispielsweise durch Wischen mit Reinigungsflüssigkeit wie Isopropanol oder auch nur Einsprühen oder Überspülen mit einer solchen Reinigungsflüssigkeit sterilisierbar sind. Über diese Reinigungs- und Sterilisationsschritte kann sichergestellt werden, dass mit dem Multi-Connector-Port 100 das Befüllen von Gefäßen auch außerhalb steriler Arbeitsumgebungen steril möglich ist. Dies senkt die Kosten für die Arbeitsschritte und erleichtert gleichzeitig das Arbeiten bei Probenentnahme oder - zugabe, die somit auch örtlich flexibel stattfinden können. Als weitere, hier nicht gezeigte Schutzmaßnahme kann der Multi-Connector-Port 100 eine oder mehrere Kappen für die Zugänge 110, 120 sowie den Luftanschluss 130 umfassen, die vor einer Zugabe oder Entnahme von Medien zunächst entfernt und nach Abschluss wieder aufgesetzt werden, sodass die Zugänge und der Luftanschluss solange sie nicht verwendet werden gegen Kontamination geschützt sind.

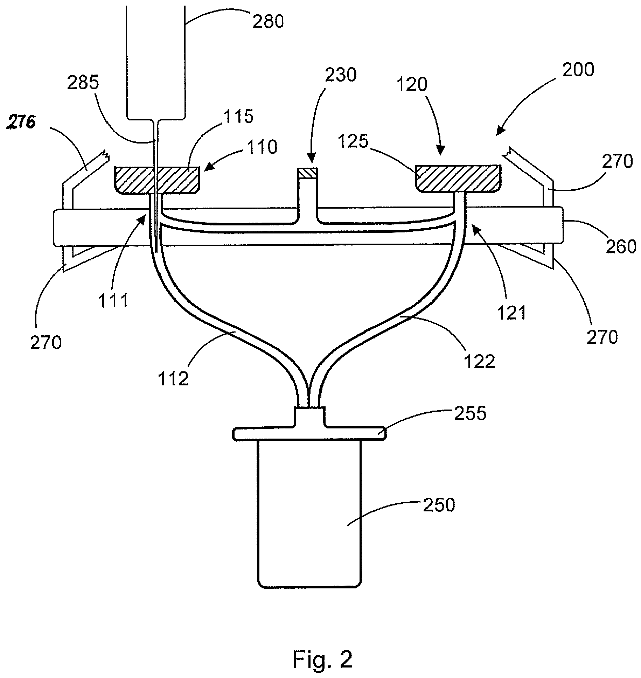

Fig. 2 zeigt eine weitere Ausführungsform eines Multi-Connector-Ports 200 gemäß dem ersten Aspekt der Erfindung. Der Multi-Connector-Port 200 ist im Wesentlichen identisch zum Multi-Connector-Port 100 aus Figur 1 aufgebaut. Daher werden nachfolgend insbesondere die weiteren Merkmale diskutiert und ansonsten auf die Beschreibung zu Figur 1 verwiesen. Identische Bauteile des Multi-Connector-Ports 200 zu denjenigen des Multi-Connector-Ports 100 sind mit gleichen Bezugszeichen versehen. Auf der Multi-Connector-Port 200 ist mit der Kopfplatte 255 eines Bioreaktors 250 verbunden. Diese ist in der gezeigten Ausführungsform allerdings in einem Liquid-Handlingsystem angeordnet, über das automatisierte Zugaben und Entnahmen realisierbar sind. Der Multi-Connector-Port 200 weist daher in der gezeigten Ausführungsform eine Fixiervorrichtung 260 auf, die hier über Klammern 270, die zum Handlingsystem gehören, mit dem Handlingsystem verbunden und fixiert sind, sodass die Zugänge 110, 120 des Multi-Connector-Ports 200 von einem Handlingroboter einfach und exakt anfahrbar sind. Exemplarisch ist hier gezeigt, wie die Spitze 285 eines Handlingroboters 280 die selbstschließende Membran 115 des Zugangs 110 penetriert. Wie zu sehen ist, dringt die hier als Hohlnadel ausgebildete Spitze 285 durch die Membran in das Verbindungsstück ein, sodass Flüssigkeit in das Verbindungsstück 110 und deren anschließende Leitung 112 abgegeben werden kann und darüber den Bioreaktor 250 erreicht. Der Multi-Connector-Port 200 weist einen Luftanschluss 230, der über ein Schraubgewinde zum Anschluss einer Leitung für sterile Luft ausgebildet ist. Über diesen Anschluss kann ein am jeweiligen Arbeitsplatz bereits vorhandenes Reservoir oder eine Leitung für sterile Luft verwendet werden, um den Multi-Connector-Port 200 zu spülen und etwaige Reste aus Zugängen, Verbindungsstücken oder Leitungen in den angeschlossenen Bioreaktor 250 zu drücken. Der Multi-Connector-Port 200 teilt ansonsten die Vorteile, die in Bezug auf den Multi-Connector-Port 100 in Figur 1 beschrieben wurden.

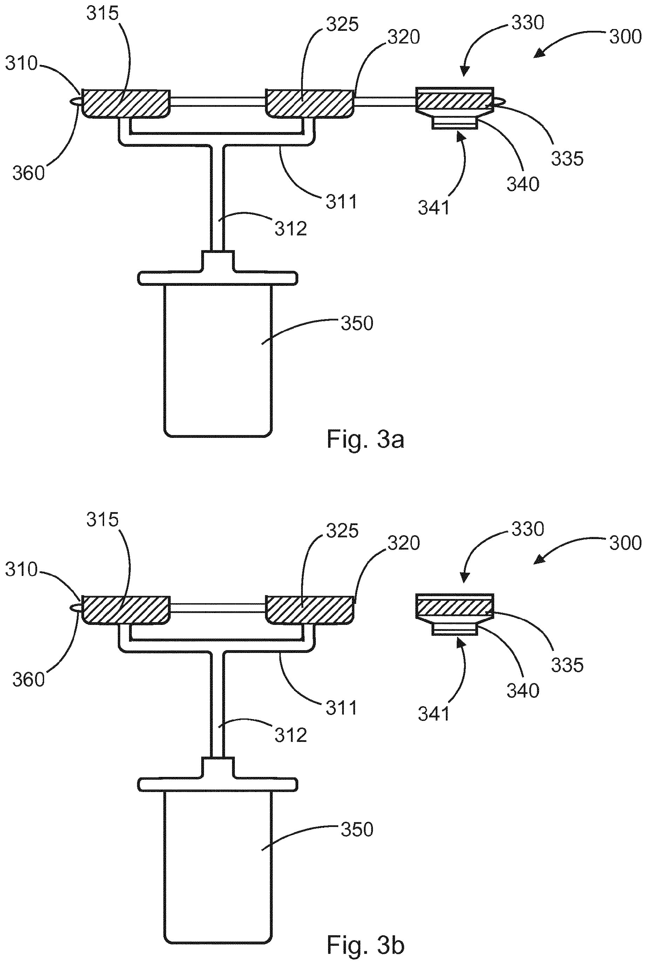

Fig. 3a zeigt eine weitere Ausführungsform eines Multi-Connector-Ports 300 gemäß dem ersten Aspekt der Erfindung. Die hier gezeigte Ausführungsform unterscheidet sich von derjenigen in den Figuren 1 und 2 dadurch, dass in der gezeigten Ausführungsform nur ein Verbindungsstück 311 vorhanden ist, an dem zwei Zugänge 310, 320 mit jeweils einer selbstschließenden Membran 315, 325 angeordnet sind und dass der Luftanschluss 330 mit der weiteren selbstschließenden Membran 335 und dem sterilen Filter 340 hier nicht fluidisch mit dem Verbindungsstück 311 verbunden ist. In der gezeigten Ausführungsform hat der sterile Filter 340 auf seiner der weiteren selbstschließenden Membran abgewandten Seite 341 Kontakt zur Außenluft. Wird nun durch die selbstschließende Membran 330 eine Spitze von oben eingeführt, so kann über diese Luft durch den sterilen Filter 340 angesaugt und in ein mit der Spitze verbundenes Reservoir gegeben werden. Hernach kann die Spitze in einen der Zugänge 310, 320 eingebracht werden und damit Luft in Verbindungsstück 311 und Leitung 312 appliziert werden, so dass in Zugang, Verbindungsstück oder Leitung befindliches Medium aus einem vorherigen Befüll- oder Entnahmevorgang in das hier verbundene Reaktionsgefäß 350 gedrückt wird. Der Multi-Connector-Port 300 weist auch einen Luftanschluss für sterile Luft 330 auf, der eine weitere selbstschließende Membran 335 sowie einen sterilen Filter 340 aufweist. Des Weiteren weist der Multi-Connector-Port 300 eine Fixiervorrichtung 360 für eine Integration in ein Handlingsystem auf. Die gezeigte Ausführungsform kann insbesondere eingesetzt werden bei Gefäßen oder Einsatzzwecken, bei den für den Multi-Connector-Port 300 wenig Platz vorhanden ist und es aufgrund des Einsatzes nicht zu problematischen Kontaminationen bei einer gemeinsamen Leitung kommen kann oder beispielsweise nur Zugaben oder nur Entnahmen im Verwendungszweck vorgesehen sind. Zur weiteren Reduktion des Platzbedarfes kann auch nur ein Zugang, beispielsweise der Zugang 310, vorgesehen sein, so dass der Multi-Connector-Port 300 auf engstem Raum realisiert sein kann.

Die in Fig. 3b gezeigte Ausführungsform unterscheidet sich von derjenigen in Fig.3a lediglich dadurch, dass der Luftanschluss 330 nicht über die Fixiervorrichtung 360 mit Zugängen 310, 320 verbunden ist. Der Luftanschluss 330 ist hier separat ausgebildet, dies ist insbesondere vorteilhaft in (hier nicht gezeigten) Ausführungsformen des Multi-Connector-Ports, die eine Vielzahl von Zugängen aufweisen, die alle mit Hilfe des einen Luftanschlusses 330 spülbar sind. Diese Ausführungsformen des Multi-Connector-Ports erlauben den gleichzeitigen Anschluss einer Vielzahl von Bioreaktoren.

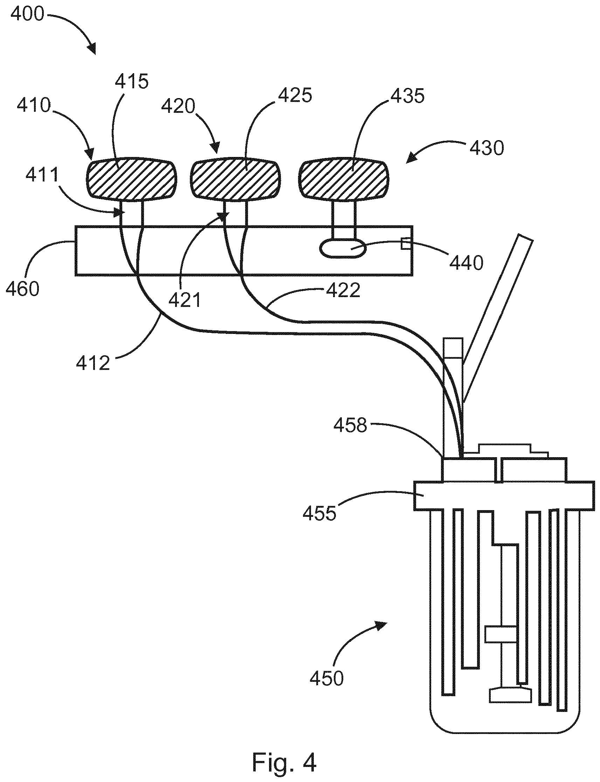

Fig. 4 zeigt eine weitere Ausführungsform eines Multi-Connector-Ports 400 gemäß dem ersten Aspekt der Erfindung. In der gezeigten Ausführungsform sind die Zugänge 410, 420 voneinander vollständig getrennt geführt. An jedem Zugang 410, 420 ist ein Verbindungsstück 411, 421 angeordnet, an dem auch jeweils eine Leitung 412, 422 angeordnet ist. Jeder der Zugänge 410, 420 weist eine selbstschließende Membran 415, 425, hier in Form eines durchstechbaren Silikonseptums auf. Bevorzugt ist der Zugang 410 für die Zugabe und der Zugang 420 für die Entnahme vorgesehen, so dass es nicht zu Verfälschungen kommt. Der Multi-Connector-Port 400 weist außerdem eine Fixiervorrichtung 460 auf, über die der Multi-Connector-Port 400 einfach an ein Handlingsystem angeschlossen werden kann. Die Fixiervorrichtung 460 verbindet gleichzeitig die Zugänge 410, 420 und den Luftanschluss 430 mechanisch. Dieser ist hier wie bereits im Hinblick auf den Multi-Connector-Port 300 und den dortigen Luftanschluss 330 näher beschrieben, mit einem sterilen Filter 440 und einer weiteren selbstschließenden Membran ausgestattet, so dass über den Luftanschluss 430 Umgebungsluft angesaugt und im Filter 440 sterilisiert werden kann und dann über eine Spitze mit angeschlossenem Reservoir zur Spülung in einen der zuvor genutzten Zugänge gegeben werden kann. Die Leitungen 412, 422 sind in der gezeigten Ausführungsform in einen Anschluss 456 in der Kopfplatte 455 eines Bioreaktors 450 eingesteckt, der über den Multi-Connector-Port 400 befüllt werden kann und aus dem Proben entnommen werden können.

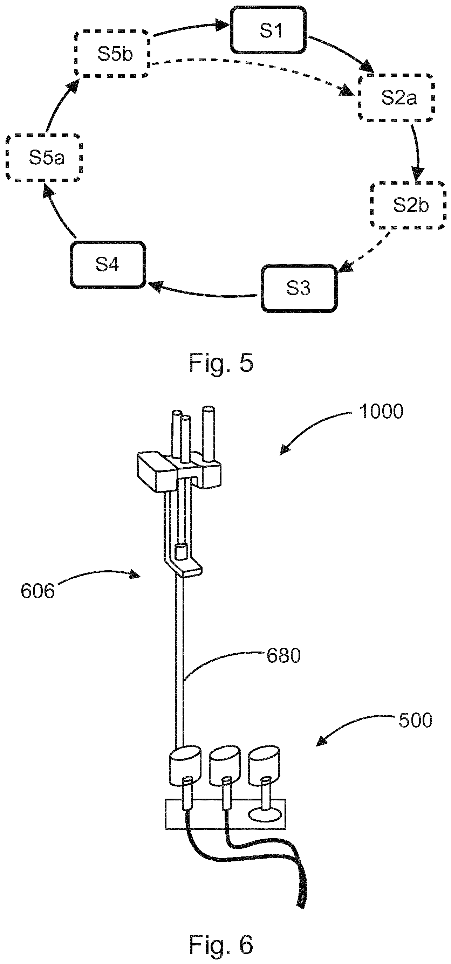

Fig. 5 zeigt eine Ausführungsform eines Verfahrens gemäß dem zweiten Aspekt der Erfindung zum Befüllen oder Entnehmen über einen Multi-Connector-Port. Vorbereitend wird in Schritt S1 die mindestens eine Leitung des Multi-Connector-Ports mit einem Gefäß, insbesondere mit einem Bioreaktor, verbunden. Die Verbindung kann dabei im einfachsten Fall durch Einbringen der beispielsweise als Schlauch ausgebildeten Leitung in eine Membran oder ein Septum beispielsweise in einer Kopfplatte eines Bioreaktors erfolgen. Erfolgt der Einsatz des Multi-Connector-Ports im Zusammenspiel mit einem Handlingsystem so erfolgt in diesem Schritt auch die exakte Positionierung und optional eine Fixierung in Bezug auf das Handlingsystem, sodass der Multi-Connector-Port von Spitzen und sonstigen Bestandteilen des Handlingsystems einfach exakt angefahren werden kann.

Im optionalen Schritt S2a wird eine über dem mindestens einen Zugang oder dem Luftanschluss angeordnete Kappe entfernt, optional wird zuvor die Kappe beispielsweise mit einem Reinigungs- und/oder Desinfektionsmittel gereinigt. In Schritt S2b wird dann eine Außenfläche des mindestens einen Zugangs und/oder eine Außenfläche des Luftanschlusses reinigt. Dies erfolgt bevorzugt durch Spülen, Besprühen oder Abwischen mit einem Reinigungsmittel und/oder Desinfektionsmittel, wie beispielsweise Isopropanol. Wird der Multi-Connector-Port in ein Handlingsystem integriert, kann dieser Schritt insbesondere automatisiert vom Handlingsystem durchgeführt werden

In Schritt S3 wird die selbstschließende Membran des wenigstens einen Zugangs mit einer Spitze, insbesondere in Form einer Hohlnadel, einer Pipettenspitze oder eines männlichen Verbindungsstücks penetriert und über die Spitze entweder ein Medium in das Gefäß gegeben oder eine Probe oder ein Medium entnommen.

In Schritt S4 wird sterile Luft in den zuvor genutzten Zugang des Multi-Connector-Ports appliziert. Dies kann über eine fluidische Verbindung zwischen Luftanschluss und Zugang erfolgen oder in einer Ausführungsform die folgenden Unterschritte beinhalten: Penetrieren der weiteren selbstschließenden Membran des Luftanschlusses mit einer Spitze; Ansaugen von Umgebungsluft durch den sterilen Filter über die Spitze in ein mit der Spitze verbundenes Reservoir; Entfernen der Spitze aus der weiteren selbstschließenden Membran, Penetrieren der selbstschließenden Membran des mindestens einen Zugangs des Multi-Connector-Ports mit der Spitze und schließlich Applizieren von Luft aus dem Reservoir in den Zugang.

In einem optionalen Schritt S5a erfolgt dann wiederum eine Reinigung, beispielsweise durch Spülen, Sprühen oder Abwischen mit einer Reinigungsflüssigkeit und schließlich soweit Kappen verwendet werden, werden diese in Schritt S5b wiederaufgesetzt und optional die Kappen mit Desinfektionsmittel besprüht.

Erfolgt die weitere Verwendung des Multi-Connector-Ports an gleicher Stelle und mit gleichem Reaktionsgefäß, so folgt dann wieder der optionale Schritt S2a. Ansonsten erfolgt wiederum eine Positionierung des Multi-Connector-Ports oder ein Verbinden mit einem weiteren Reaktionsgefäß in Schritt S1.

Fig. 6 zeigt eine Ausführungsform eines Systems 1000 gemäß dem dritten Aspekt der Erfindung Das System 1000 umfasst in der gezeigten Ausführungsform eine Multi-Connector 500 umfassend einen Luftanschluss für sterile Luft sowie mindestens einen an einem Verbindungsstück angeordneten Zugang und mindestens eine am Verbindungsstück angeordnete Leitung, wobei der Zugang eine selbstschließende Membran umfasst, wie er beispielsweise in den Figuren 1 bis 4 detailliert beschrieben ist. Darüber hinaus umfasst das System 1000 ein Handlingsystem 600 mit mindestens einer Spitze 680, hier in Form einer Hohlnadel. In der gezeigten Darstellung ist das Handlingsystem nur ausschnittsweise dargestellt. Mit dem System 1000 können nun bei Anschluss des Multi-Connector-Ports 500 an beispielweise einen Bioreaktor Entnahmen oder Zugaben aus oder an diesen automatisiert in nicht-steriler Umgebung erfolgen.

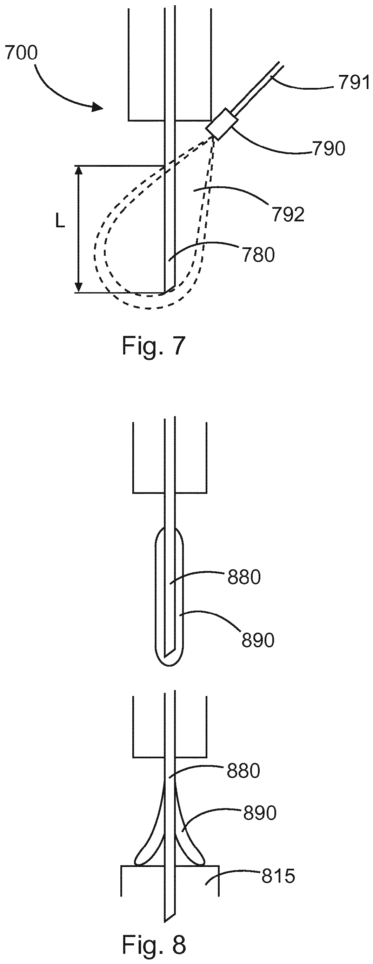

Fig. 7 zeigt in einer Detailansicht Teile einer Ausführungsform eines Systems gemäß dem dritten Aspekt der Erfindung. Hier ist im Detail eine Spitze 780 eines nicht weiter dargestellten Handlingsystems 700 als Teil des Systems dargestellt, wobei das System eine Düseneinrichtung 790 mit einer Düse zur Umspülung der Spitze 780 mit steriler Luft umfasst. Die Düse ist mit einem Anschluss für sterile Luft 791 verbunden und so auf die Spitze gerichtet, dass diese in einer Länge I von steriler Luft umspült wird und keinen Kontakt zur Umgebungsluft hat. Es wird über die Düseneinrichtung 790 somit ein steriler Luftvorhang 792 um die Spitze etabliert. Die Umspülung mit steriler Luft kann dauerhaft erfolgen oder zumindest während eines Eindringens der Spitze bis zur vollständigen Penetration und so lange Spitze nicht penetriert.

Fig. 8 zeigt in einer Detailansicht Teile einer Ausführungsform eines Systems gemäß dem dritten Aspekt der Erfindung in zwei Zuständen. Das hier in Teilen dargestellte System umfasst eine Kapselungsvorrichtung in Form einer selbstschließenden Ummantelung 890, die hier als Silikonkappe mit einem Kreuzschlitz ausgeführt ist, dabei weist die Silikonkappe an einem Ende, das von einem offenen Ende der Spitze 880 abgewandt ist eine Öffnung für die Aufnahme der Spitze auf und an ihrem entgegengesetzten Ende, das dem offenen Ende der Spitze zugewandt ist den Kreuzschlitz. In der oberen Figur ist die Spitze 880 des Handlingsystems des Systems frei und von der selbstschließenden Ummantelung gegen die Umgebungsluft gekapselt. Wird nun, wie in der unteren Figur dargestellt, die Spitze 880 beispielsweise in eine selbstschließende Membran 815 gedrückt, so wird bei Kontakt von selbstschließender Ummantelung 890 und selbstschließender Membran 815 die selbstschließende Ummantelung aufgedrückt, so dass ein Teil der Spitze 880 in die selbstschließende Membran eindringen kann, während die weiteren von der Ummantelung 890 umgebenen Teile der Spitze 880 von dieser geschützt bleiben. Die Ummantelung 890 schließt dann zusammen mit der selbstschließenden Membran 815 die Spitze gegen die Umgebungsluft ab. Sobald die Spitze 880 zurückgezogen wird, legt sich die Ummantelung 890 wieder um die Spitze 880.

## Patentansprüche

1. Multi-Connector-Port umfassend einen Luftanschluss für sterile Luft sowie mindestens einen an einem Verbindungsstück angeordneten Zugang und mindestens eine am Verbindungsstück angeordnete Leitung, wobei der Zugang eine selbstschließende Membran umfasst.

2. Multi-Connector-Port nach Anspruch 1, bei dem der Luftanschluss eine weitere selbstschließende Membran und einen sterilen Filter umfasst, insbesondere einen sterilen Filter mit einem Porendurchmesser kleiner oder gleich 0,22 µm.

3. Multi-Connector-Port nach Anspruch 2, bei dem eine der selbstschließenden Membran abgewandte Seite des sterilen Filters Kontakt zur Außenluft hat.

4. Multi-Connector-Port nach Anspruch 1 bis 3, wobei der Luftanschluss derart ausgebildet ist, dass über ihn sterile Luft in eine Spitze gezogen werden kann.

5. Multi-Connector-Port nach Anspruch 1 oder 2, wobei der mindestens eine Zugang, der Luftanschluss und die mindestens eine Leitung miteinander über das mindestens eine Verbindungsstück fluidisch verbunden sind.

6. Multi-Connector-Port nach einem der vorstehenden Ansprüche mit genau zwei Zugängen, bei dem ein Zugang für eine Zugabe von Medien und ein Zugang für eine Entnahme von Medien ausgebildet ist.

7. Multi-Connector-Port nach einem der vorstehenden Ansprüche aufweisend eine Fixiervorrichtung, insbesondere zur Verbindung mit einem Handlingsystem, insbesondere einem Handlingroboter, aufweist.

8. Multi-Connector-Port nach einem der vorstehenden Ansprüche, der sterilisierbar ausgeführt ist, wobei die Sterilisation bevorzugt über Autoklavieren, Bestrahlung oder mit Ethylenoxid erfolgen kann.

9. Multi-Connector-Port nach einem der vorstehenden Ansprüche umfassend eine Kappe zum Abdecken des mindestens einen Zugangs und/oder des Luftanschlusses.

10. Multi-Connector-Port nach einem der vorstehenden Ansprüche, bei dem die selbstschließende Membran als durchstechbares Septum ausgebildet ist oder bei dem der Zugang als nadelfreies Membranventil umfassend die selbstschließende Membran ausgebildet ist.

11. Verfahren zum Befüllen oder Entnehmen über einen Multi-Connector-Port nach einem der vorstehenden Ansprüche umfassend die Schritte
- Verbinden der mindestens einen Leitung des Multi-Connector-Ports mit einem Gefäß, insbesondere einem Bioreaktor,
- Reinigen einer Außenfläche des mindestens einen Zugangs und/oder einer Außenfläche des Luftanschlusses, insbesondere durch Abwischen oder Spülen mit einem Reinigungsmittel umfassend Isopropanol,
- Penetrieren der selbstschließenden Membran des mindestens einen Zugangs mit einer Spitze, insbesondere in Form einer Hohlnadel, einer Pipettenspitze oder einem männlichen Verbindungsstück,
- Zugeben eines Mediums in das Gefäß oder Absaugen eines Mediums aus dem Gefäß über die Spitze,
- Entfernen der Spitze,
- Applikation von steriler Luft.

12. Verfahren nach Anspruch 11, wobei die Applikation von steriler Luft folgende Schritte umfasst:
- Penetrieren der weiteren selbstschließenden Membran des Luftanschlusses mit einer Spitze
- Ansaugen von Umgebungsluft durch den sterilen Filter über die Spitze in ein mit der Spitze verbundenes Reservoir
- Entfernen der Spitze aus der weiteren selbstschließenden Membran
- Penetrieren der selbstschließenden Membran des mindestens einen Zugangs des Multi-Connector-Ports mit der Spitze
- Applizieren von Luft aus dem Reservoir in den Zugang.

13. Verfahren nach Anspruch 11 oder 12, bei dem nach der Applikation steriler Luft eine abschließende Reinigung der Außenfläche des mindestens einen Zugangs und/oder der Außenfläche des Luftanschlusses erfolgt.

14. Verfahren nach einem der Ansprüche 11 bis 13, bei dem der Multi-Connector-Port mindestens eine Kappe umfasst und diese vor dem Reinigen abgenommen wird und/oder nach der abschließenden Reinigung aufgesetzt wird.

15. Verfahren nach einem der Ansprüche 11 bis 14, bei dem die Spitze Teil eines Handlingsystems ist und das Verbindungsstück eine Fixiervorrichtung aufweist und bei dem der Multi-Connector-Port vor dem Reinigen in Relation zum Handlingsystem über die Fixiervorrichtung exakt positioniert wird.

16. System umfassend einen Multi-Connector-Port nach einem der Ansprüche 1 bis 10 sowie ein Handlingsystem mit mindestens einer Spitze, insbesondere in Form einer Hohlnadel, einer Pipettenspitze oder einem männlichen Verbindungsstück.

17. System nach Anspruch 16 weiter umfassend eine Kapseleinrichtung zur Kapselung der Spitze gegen Umgebungsluft, insbesondere eine Düseneinrichtung zur Umspülung der Spitze mit steriler Luft oder eine selbstschließende Ummantelung.
